# DEMANDE DE BREVET EUROPEEN

(11) **EP 0 800 815 A2**
(43) Date de publication de la demande: **15.10.1997**
(21) Numéro de dépôt: 97400727.0
(22) Date de dépôt: 28.03.1997
(51) Int. Cl.: A61K 7/06

(54) **Utilisation d'au moins un inhibiteur de lipoxygénase et d'au moins un inhibiteur de cyclo-oxygénase pour modifier la pousse des poils et/ou des cheveux**

(30) Priorité: 17.04.1996 FR 9604795
(71) Demandeur: L'OREAL, 75008 Paris (FR)
(72) Inventeur: Duranton, Albert, 75018 Paris (FR)
(74) Mandataire: Tezier Herman, Béatrice

(57) **Abrégé**

L'invention concerne l'utilisation, dans une composition cosmétique ou pour la préparation d'un médicament pour inhiber la pousse des poils et/ou des cheveux, d'au moins un inhibiteur de lipoxygénase et d'au moins un inhibiteur de cyclo-oxygénase.

L'invention concerne également des compositions comprenant au moins un inhibiteur de lipoxygénase et au moins un inhibiteur de cyclo-oxygénase.

## Description

La présente invention concerne, dans son aspect le plus général, l'utilisation dans une composition cosmétique ou pour la préparation d'un médicament pour modifier la pousse des poils et/ou des cheveux, d'au moins un inhibiteur de lipoxygénase et d'au moins un inhibiteur de cyclo-oxygénase.

Plus particulièrement, elle concerne l'utilisation dans une composition cosmétique ou pour la préparation d'un médicament pour soit favoriser la chute des poils et/ou cheveux, soit diminuer ou empêcher leur pousse, d'au moins un inhibiteur de lipoxygénase et d'au moins un inhibiteur de cyclo-oxygénase.

Elle concerne également divers types des compositions comprenant au moins un inhibiteur de lipoxygénase et au moins un inhibiteur de cyclo-oxygénase.

On sait que certains acides gras polyinsaturés, en particulier ceux présentant 20 atomes de carbone, tels que l'acide arachidonique, l'acide dihomogammalinolénique ou bien encore l'acide éicosapentaénoïque, peuvent être transformés in vivo, sous l'action de certaines enzymes spécifiques contenues dans les cellules vivantes, en particulier les cellules épithéliales, en certains autres composés de type éicosanoïdes utiles à l'organisme.

Ainsi, il est connu que les enzymes dites cyclo-oxygénases génèrent, à partir des différents acides gras mentionnés ci-dessus, des éicosanoïdes de type prostaglandines et tromboxanes, et que les enzymes dites lipoxygénases sont, quant à elles, responsables de la formation des éicosanoïdes de type leukotriènes et autres acides acycliques hydroxylés à 20 atomes de carbone. Un même acide gras polyinsaturé donné (ou substrat) pourra donner lieu, selon la nature de l'enzyme avec laquelle il aura réagi en premier, à la formation de plusieurs métabolites différents, à savoir par exemple des prostaglandines et des leukotriènes.

Les acides gras polyinsaturés notamment en C₂₀ (matières premières réactives) appelés à être métabolisés sous l'action spécifique des enzymes cyclo-oxygénases et lipoxygénases sont généralement apportés à l'organisme par l'intermédiaire de certains aliments, en particulier de certaines huiles naturelles d'origine animale ou végétale, cet apport pouvant alors se faire soit sous une forme directe (c'est le cas par exemple de l'acide arachidonique, qui est présent tel quel dans les blancs d'oeufs), soit indirectement sous la forme de composés précurseurs (composés que l'on appelle aussi "acides gras essentiels", qui sont eux-mêmes des acides gras insaturés, généralement en C₁₈-C₂₂, tels que les acides linoléïque, α-linolénique et γ-linolénique) qui seront transformés par le métabolisme humain, selon des mécanismes complexes qu'il n'est pas nécessaire de détailler ici, en substrats convenables (c'est à dire métabolisables) pour cyclo-oxygénases et lipoxygénases.
On sait que les transformations enzymatiques ci-dessus exposées et les différents produits de réaction qui en résultent ont une influence non négligeable sur les mécanismes de pousse du poil et/ou du cheveu.
A ce titre la demanderesse a montré qu'en privilégiant l'une ou l'autre des deux voies enzymatiques, cyclo-oxygénases ou lipoxygénases, au sein des cellules de la peau et/ou du cuir chevelu, on pouvait modifier de manière substantielle la pousse des poils et/ou des cheveux. Ceci a fait l'objet de la demande de brevet EP 94-402055.
Essentiellement, dans cette demande de brevet, la demanderesse préconisait de favoriser l'une des voies par rapport à l'autre par l'administration de combinaison de composés associant un inhibiteur de l'une des voies à un stimulateur de l'autre voie.

Dans les demandes de brevet WO/94/27563 et WO/94/27586, il est proposé de réduire, voir d'inhiber la pousse des poils ou des cheveux, par l'administration de quantité efficace soit d'inhibiteur de lipoxygénase, soit d'inhibiteur de cyclo-oxygénase. Les quantités d'inhibiteurs qui sont préconisées dans ces demandes vont de 1% à 30% en poids, voire plus. L'expérience montre que les effets commencent à être mesurables à partir de 5% et ne présentent de réels intérêts qu'à des doses supérieures à 10 ou 15%, si ce n'est 20%.

Sans dénigrer aucunement l'intérêt de ces demandes de brevets, il faut souligner que les composés généralement utilisés peuvent présenter des effets secondaires et peuvent être parfois toxiques au dessus de certaines doses. Ceci a pour conséquence de rendre leur utilisation délicate, voire totalement impossible, en particulier dans des applications cosmétiques.

C'est pour pallier à ces inconvénients que la demanderesse a cherché à améliorer l'efficacité des inhibiteurs de lipoxygénase et de cyclo-oxygénase, en particulier afin de pouvoir les utiliser à des doses très largement inférieures aux doses pour lesquelles les effets secondaires indésirables pourraient apparaître.

De manière surprenante et inattendue, la demanderesse a découvert que l'association d'un inhibiteur de lipoxygénase et d'un inhibiteur de cyclo-oxygénase a le pouvoir d'inhiber la pousse des poils et/ou cheveux, qui plus est à des doses de chacun des inhibiteurs pour lesquelles ceux-ci pris individuellement ne présentent aucune activité ou au contraire présentent une activité inverse.

Cette découverte est à la base de la présente invention.

Dans l'exposé qui suit de la présente invention, on entend désigner :
- par "inhibiteur" de lipoxygénase ou de cyclo-oxygénase, toute substance permettant, in vivo, de limiter ou d'inhiber totalement l'activité enzymatique respectivement des lipoxygénases ou des cyclo-oxygénases,
- par "voie topique", toute technique d'administration d'un produit par application directe de ce dernier sur une partie superficielle (ou externe) du corps, telle que peau, cheveux et autres,
- par "voie systémique", toute technique d'administration d'un produit par une voie autre que topique, par exemple orale et/ou parentérale.

Ainsi, selon un premier aspect de la présente invention, il est maintenant proposé l'utilisation, dans une composition cosmétique ou pour la préparation d'un médicament pour favoriser la chute des poils et/ou des cheveux ou à ralentir et/ou empêcher leur pousse, d'au moins un inhibiteur de lipoxygénase et d'au moins un inhibiteur de cyclo-oxygénase.

Il est bien entendu possible d'utiliser selon l'invention au moins un composé qui se révélerait être à la fois un inhibiteur de lipoxygénase et un inhibiteur de cyclo-oxygénase.

Selon un autre aspect de l'invention, il est proposé des compositions, nouvelles en soi, comprenant au moins deux composés distincts, l'un étant au moins un inhibiteur de lipoxygénase et l'autre étant au moins un inhibiteur de cyclo-oxygénase.

Selon encore un autre aspect de la présente invention, il est également proposé des dispositifs à plusieurs compartiments ou "kits", caractérisés par le fait qu'ils comprennent dans un premier compartiment un ou plusieurs inhibiteurs de lipoxygénase et, dans un deuxième compartiment, un ou plusieurs inhibiteurs de cyclo-oxygénase, les compositions contenues dans lesdits premier et second compartiments étant ici considérées comme compositions de combinaison pour une utilisation simultanée, séparée ou étalée dans le temps plus particulièrement destinée à modifier la pousse des poils et/ou des cheveux.

Mais d'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

On commencera par détailler, dans leur nature, les différents produits et compositions utilisables dans le cadre de la présente invention.

Le caractère inhibiteur d'une substance donnée vis-à-vis des lipoxygénases ou des cyclo-oxygénases peut être classiquement déterminé par l'homme de l'art au moyen notamment de tests biochimiques usuels, généralement basés sur des méthodes d'analyses chromatographiques. Ainsi, ces activités peuvent être par exemple explorées avec les techniques suivantes (liste non limitative) ou avec toute autre technique standardisée :
- activités vis-à-vis des 5, 12 et 15 lipoxygénases : on peut ici citer la méthode consistant à utiliser un matériel biologique (polynucléaires humains, cheveux) incubé en présence d'acide arachidonique C14 ou d'acide linoléique C14 ; les hydroxyacides formés sont extraits, séparés par chromatographie sur couche mince ou chromatographie HPLC (Vanderhoeck J.Y et Bailey J.M. dans J. Biol. Chem. 1984 259 pp 6752-6761 ; Huang M. et al., dans Cancer Res. 51 pp 813-819, 1991 ; Baer A.N et Green F.A. dans J. Lipids Res. 1993 34 pp 1505-1514 ; Ziboh V.A. et al. dans J. invest. Dermatol. 83 pp 248-251, 1984).
- activités vis-à-vis de la 5 lipoxygénase: on peut ici citer les méthodes spectrophotométriques décrites par Aharony D. et Stein R.L. dans J. Biol. Chem. 1986 261 pp 11512-11517, et par McMillan R.M. et al. dans Biochim. Biophys. Acta 1989 1005 pp 170-176.
- activités vis-à-vis des cyclo-oxygénases: on peut ici citer la méthode reposant sur l'utilisation d'un matériel biologique (épiderme) incubé en présence d'acide arachidonique C14 ; les hydroxyacides formés sont extraits, séparés par chromatographie HPLC (Huang M. et al. dans Cancer Res. 51 pp 813-819, 1991) ou identifiés par radioimmunoessais (Lysz T.W. et Needleman P. J, dans Neurochim. 38 pp 1111-1117, 1982). On peut également mentionner le test décrit dans l'article "Nitric Oxide Activates Cyclo-oxygénase Enzymes, par D. SALVAMENI et al., Proc. Natl. Sci. USA, Vol.90, pp 7240-7244, August 1993".

Les inhibiteurs de lipoxygénase peuvent être notamment choisis parmi les inhibiteurs redox et non redox, les précurseurs d'inhibiteurs redox, les agents anti-oxydants, les agents chélateurs du fer, les composés imidazolés, les phénothiazines, les dérivés du benzopyrane, ainsi que parmi certains éicosanoïdes.

L'inhibiteur redox peut être choisi parmi les dérivés des butanes catécholiques (US 5008294, US 4708964 et US 4880637), tels que l'acide nordihydroguaiarétique (NDGA), ou l'un de ses énantiomères tel que le masoprocol.

L'inhibiteur redox peut aussi être choisi parmi la phénidone, le lonapalen, les indazolinones, le naphazatrom, le benzofuranol, l'alkylhydroylamine, les composés de formules suivantes :

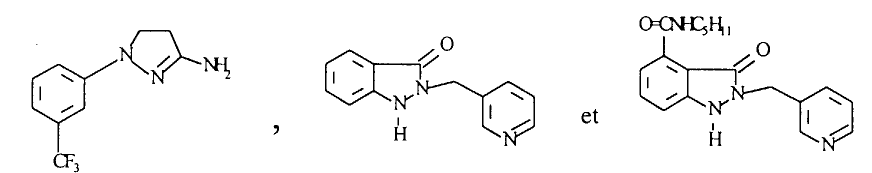

Les inhibiteurs non redox peuvent être choisis parmi les hydroxythiazoles, les méthoxyalkylthiazoles, les benzopyranes et leurs dérivés, le méthoxytétrahydropyrane, les acides boswelliques et leurs dérivés acétylés, les acides quinolinméthoxyphénylacétiques substitués par des radicaux cycloalkyles.

L'antioxydant peut être choisi parmi les phénols, le propylgalate, les flavonoïdes et les composés naturels contenant de tels flavonoïdes (Ginkgo Biloba).

Parmi les flavonoïdes utilisables selon l'invention, on peut citer les dérivés hydroxylés des flavones tels que le flavonol, la dihydroquercétine, la lutéoline, la galangine, l'orobol. On peut également citer les dérivés du chalcone tel que le 4,2',4'-trihydroxychalcone, les orthoaminophénols, les N-hydroxyurées, les benzofuranols, l'ebselen et les agents susceptibles d'accroître l'activité des séléno-enzymes réductrices.

L'agent chélateur du fer peut être choisi parmi les acides hydroxamiques et leurs dérivés, les N-hydroxyurées, le 2-benzyl-1-naphtol, les catéchols, les hydroxylamines, le carnosol trolox C, le catéchol, le naphtol, la sulfasalazine, le zyleuton, l'acide 5-hydroxyanthranilique et les acides 4-(omégaaryllalkyl)phénylalcanoïques.

Les composés imidazolés peuvent être choisis parmi le kétoconazole, l'itraconazole.

Parmi les éicosanoïdes inhibiteurs de lipoxygénase, on peut citer les acides octa-décatétraénoïque, éicosatétraénoïq ue, docosapenténoïque, eicosahexaénoïque, docosahexaénoïque, et leurs différents esters, de même que divers autres éicosanoïdes, éventuellement sous forme d'esters, tels que PGE1 (prostaglandine E1), PGA2 (prostaglandine A2), le viprostol, les acides 15-monohydroxyéicosatétraénoïque, 15-monohydroxyéicosatriénoïque et 15-monohydroxyéicosapentaénoïque, les leukotriènes B5, C5 et D5.

A titre d'autres composés divers pouvant inhiber les lipoxygénases, on peut également citer les produits interférant avec les flux calciques, en particulier les phénothiazines et les diphénylbutylamines, le vérapamil, le fuscoside, le curcumin, l'acide chlorogénique, l'acide caféique, l'acide 5,8,11,14-éicosatétrayénoïque (ETYA), l'hydroxyphénylrétinamide, le lonapalène, l'esculin, le diéthylcarbamazine, la phénantroline, la baicaléine, le proxicromil, les thioéthers et en particulier le sulfure de diallyle et le sulfure de di-(1 propènyl).

Les inhibiteurs de cyclo-oxygénase peuvent être notamment choisis parmi les agents anti-inflammatoires non-stéroïdiens tels que les dérivés arylcarboxyliques, les dérivés pyrazolés, les dérivés de l'oxicam, les dérivés de l'acide nicotinique.
Comme inhibiteurs de cyclo-oxygénase on peut également utiliser dans l'invention l'acide méclofenamique (5659, Merck index, 11ième édition), l'acide méfénamique (5680, Merck index, 11ième édition), le carprofen (1870, Merck index, 11ième édition), le diclofenac (3071, Merck index, 11ième édition), le diflunisal (3130, Merck index, 11ième édition), le fenbufen (3906, Merck index, 11ième édition), le fenoprofen (3926, Merck index, 11ième édition), l'ibuprofen (4812, Merck index, 11ième édition), l'indométhacine (4874, Merck index, 11ième édition), le ketoprofen (5184, Merck index, 11ième édition), la nabumetone (6258, Merck index, 11ième édition), le naproxen (6337, Merck index, 11ième édition), le sulindac (8961, Merck index, 11ième édition), le tenoxicam (9080, Merck index, 11ième édition), la tolmetine (9441, Merck index, 11ième édition), ou encore l'acide acetylsalicylique.

Enfin, parmi les substances pouvant être utilisées à la fois comme inhibiteur de lipoxygénase et inhibiteur de cyclo-oxygénase, on peut plus particulièrement citer les corticoïdes tels que la dexaméthasone ou l'hydrocortisone, les fénamates tels que le morniflumate, les dérivés de chalcone tels que le 3,4-dihydroxychalcone, ou les inhibiteurs de phospholipase A₂.

On notera enfin d'une manière générale que, dans le cadre de la présente invention, il est bien évidemment tout à fait possible d'utiliser des mélanges d'inhibiteurs pour autant bien entendu que ces mélanges restent compatibles avec l'effet recherché.

Selon l'invention, l'inhibiteur de lipoxygénase peut être utilisé en une quantité allant de 0,01 % à 5 % en poids, de préférence allant de 0,1 % à 1 % et encore plus préférentiellement allant de 0,1 % à 0,8 %.
De même, l'inhibiteur de cyclo-oxygénase peut être utilisé en une quantité allant de 0,001% à 5 % en poids et de préférence allant de 0,01 % à 0,1%.

Les compositions selon l'invention peuvent être utilisées à titre de médicament plus particulièrement destiné à favoriser la chute des poils et/ou des cheveux ou à ralentir et/ou empêcher la pousse des poils et/ou des cheveux.

D'une manière générale, on notera que tous les produits ci-dessus peuvent être classiquement conditionnés sous une forme convenant au mode d'administration ou d'application retenu pour ces derniers (lotions, shampooings, comprimés, sirops et autres).
Les compositions ou les "kits" rentrant dans le cadre de la présente invention sont plus particulièrement les suivants :
- les compositions comprenant au moins un inhibiteur de lipoxygénase et au moins un inhibiteur de cyclo-oxygénase;
- les "kits" comprenant dans un premier compartiment au moins un inhibiteur de lipoxygénase et, dans un deuxième compartiment, au moins un inhibiteur de cyclo-oxygénase.

Comme indiqué précédemment, chacune des compositions ainsi que chacun des composants rentrant dans les compartiments des kits sont classiquement conditionnés sous une forme convenant aux différents modes d'administration ou d'application envisagés pour ces derniers (lotions, shampooings, comprimés, sirops et autres). Ainsi, les compositions et les kits sont de préférence conditionnés sous une forme convenant à une application topique.

D'une manière générale, selon la présente invention, il est en fait possible de concevoir des kits de présentation contenant autant de compartiments séparés que d'inhibiteurs que l'on désire ou qu'il convient de mettre en oeuvre.

Les compositions selon l'invention, ou les kits selon l'invention, peuvent également comprendre divers additifs classiques et usuels, en particulier cosmétiques dans le cas d'applications topiques (produits capillaires notamment), choisis par exemple parmi des filtres UV, des agents épaississants, des agents de pénétration tel que l'urée, des solvants organiques tels que l'éthanol, l'isopropanol, les alkylèneglycols, des agents tensioactifs choisis parmi les tensioactifs non-ioniques tels que les alkylpolyglycosides, les tensioactifs cationiques, les tensioactifs anioniques et les tensioactifs amphotères, des colorants, des agents antipelliculaires, des parfums et des conservateurs.

Selon encore un autre aspect de la présente invention, il est également proposé un procédé de traitement non thérapeutique pour modifier la pousse des poils et/ou des cheveux, caractérisé en ce qu'il comprend une étape d'administration à l'organisme, par voie topique et/ou systémique, d'au moins un inhibiteur de lipoxygénase et d'au moins un inhibiteur de cyclo-oxygénase.

Comme indiqué précédemment, ladite administration peut se faire en combinaison de manière simultanée, séparée ou étalée dans le temps.

De manière préférentielle, ces inhibiteurs sont administrés par voie topique.

Selon un mode particulièrement préféré de mise en oeuvre du procédé selon l'invention, on applique sur la peau et/ou le cuir chevelu des compositions à usage topique contenant conjointement les inhibiteurs de lipoxygénase et de cyclo-oxygénase.

Pour obtenir des effets notables, la fréquence d'administration ou d'application des compositions selon l'invention est de l'ordre de une à deux fois par jour. A cet égard, on a noté que les quantités suffisantes en agents inhibiteurs à mettre en oeuvre dans le cadre de l'invention peuvent, généralement, rester très faibles.

La présente invention trouve des applications particulièrement utiles dans le domaine des traitements des diverses pathologies affectant la peau et/ou le cuir chevelu, en particulier l'hirsutisme.

Des exemples concrets illustrant l'invention vont maintenant être donnés.

### Exemple 1 :

Mesure de l'effet de l'association d'un inhibiteur de lipoxygénase et d'un inhibiteur de cyclo-oxygénase sur la repousse du poil.

Le principe de l'expérience consiste à utiliser des souris dont le poil est en phase inactive. Un rasage va induire la repousse du poil.

Les animaux sont traités par lots correspondant aux différentes conditions à tester.

On mesure pour chaque lot le temps de repousse et le nombre d'animaux concernés par cette repousse.

La souris C57BL6 est choisie car chez cet animal la synthèse de la mélanine n'a lieu que dans les poils en phase active de repousse. Lorsque les animaux sont rasés, ils apparaissent roses de peau. On ne conserve pour l'expérience que des animaux dont la peau après rasage apparaît totalement rose.
Les animaux sont observés quotidiennement. On note sur le lot témoin (sans traitement) le délai d'apparition des poils. On détermine ainsi un délai moyen de repousse. On détermine alors au même délai le nombre d'animaux des lots tests ayant une repousse équivalente.

On considère qu'il y a inhibition de la repousse du poil quand le délai moyen est augmenté ou quand, au même délai, le nombre d'animaux présentant un taux de repousse équivalent est diminué.

Le traitement des animaux est fait quotidiennement par voie topique à la dose de 50 µl de composition à tester sur 3 cm² pendant 35 jours.
Le lot témoin ne reçoit qu'une composition faite du véhicule (propylène glycol 22,8%, éthanol 55,1% et eau qsp 100% (en poids)).
Lot A : Témoin ;
Lot B : Indométhacine à 0,05 g/100 ml ;
Lot C : N.D.G.A à 1 g/100 ml ;
Lot D : Indométhacine à 0,05 g/100 ml + N.D.G.A à 1 g/100 ml.

Dans l'expérience le temps moyen est déterminé à 20 jours.

| | Lot A | Lot B | Lot C | Lot D |
|---|---|---|---|---|
| % d'animaux poilus à 20 jours | 55 | 45 | 87 | 10 |

Un test de chi2 relatif au nombre d'animaux donne un degré de liberté de 3,00, un chi2 de 21,03 avec une probabilité de 0,0001, indiquant que dans les conditions expérimentales l'association des 2 inhibiteurs empêche la repousse des poils de manière significative.

### Exemple 2 :

Exemples de compositions selon l'invention :

| Composition 1 : Lotion non rincée | |
|---|---|
| NDGA | 0,10 g |
| Indométhacine | 0.05 g |
| Propylène glycol | 22,80 g |
| Ethanol 95° | 55,10 g |
| Eau purifiée | qsp 100,00 g |

| Composition 2 : Lotion non rincée | |
|---|---|
| NDGA | 0,10 g |
| Ibuprofen | 0,25 g |
| Propylène glycol | 22,80 g |
| Ethanol 95° | 55,10 g |
| Eau purifiée | qsp 100,00 g |

| Composition 3 : Lotion rincée | |
|---|---|
| NDGA | 5,00 g |
| Indométhacine | 0,25 g |
| Propylène glycol | 22,80 g |
| Ethanol 95° | 55,10 g |
| Eau purifiée | qsp 100,00 g |

| Composition 4 : Lotion non rincée | |
|---|---|
| NDGA | 3,00 g |
| Acide méclofénamique | 0,25 g |
| Propylène glycol | 22,80 g |
| Ethanol 95° | 55,10 g |
| Eau purifiée | qsp 100,00 g |

| Composition 5 : Shampooing | |
|---|---|
| NDGA | 1,00 g |
| Tenoxicam | 0,25 g |
| Tensio-actif APG 300 | 15,00 g MA (= 30 g) |
| Eau purifiée | qsp 100,00 g |

| Composition 6 : Lotion non rincée | |
|---|---|
| Gingko Biloba ⁽¹⁾ | 5,00 g |
| Indométhacine | 0,25 g |
| Propylène glycol | 22,80 g |
| Ethanol 95° | 55,10 g |
| Eau purifiée | qsp 100,00 g |

| | |
|---|---|
| (1): extrait naturel riche en flavonoïdes | |

| Composition 7 : Lotion non rincée | |
|---|---|
| Kétoconazole | 0,50 g |
| Indométhacine | 0,25 g |
| Propylène glycol | 22,80 g |
| Ethanol 95° | 55,10 g |
| Eau purifiée | qsp 100,00 g |

| Composition 8 : Lotion non rincée | |
|---|---|
| Sulfure de diallyle | 5,00 g |
| Indométhacine | 0,25 g |
| Propylène glycol | 22,80 g |
| Ethanol 95° | 55,10 g |
| Eau purifiée | qsp 100,00 g |

| Composition 9 : Lotion non rincée | |
|---|---|
| Gingko Biloba | 5,00 g |
| Indométhacine | 0,25 g |
| Acide linoléique | 5,00 g |
| Propylène glycol | 22,80 g |
| Ethanol 95° | 55,10 g |
| Eau purifiée | qsp 100,00 g |

| Composition 10: Lotion non rincée | |
|---|---|
| Kétoconazole | 0,50 g |
| Kétoprofen | 0,20 g |
| Propylène glycol | 22,80 g |
| Ethanol 95° | 55,10 g |
| Eau purifiée | qsp 100,00 g |

| Composition 11 : Lotion non rincée | |
|---|---|
| Kétoconazole | 0,50 g |
| Acide acétylsalicylique | 0,25 g |
| Propylène glycol | 22,80 g |
| Ethanol 95° | 55,10 g |
| Eau purifiée | qsp 100,00 g |

| Composition 12 : Lotion non rincée | |
|---|---|
| lonapalene | 1,00 g |
| Indométhacine | 0,25 g |
| Propylène glycol | 22,80 g |
| Ethanol 95° | 55,10 g |
| Eau purifiée | qsp 100,00 g |

Toutes les compositions 1 à 12 ci-dessus ont donné de bons résultats dans le ralentissement de la pousse des poils et des cheveux.

## Revendications

1. Utilisation dans une composition cosmétique ou pour la préparation d'un médicament pour favoriser la chute des poils et/ou des cheveux ou pour ralentir et/ou empêcher la pousse des poils et/ou des cheveux, d'au moins un inhibiteur de lipoxygénase et d'au moins un inhibiteur de cyclo-oxygénase.

2. Utilisation selon la revendication 1, caractérisé en ce que l'on utilise au moins une substance qui est à la fois un inhibiteur de lipoxygénase et un inhibiteur de cyclo-oxygénase.

3. Utilisation selon l'une quelconque des revendications précédentes, caractérisé en ce que les inhibiteurs de lipoxygénase et/ou de cyclo-oxygénase sont administrés par voie topique.

4. Utilisation selon l'une quelconque des revendications précédentes, caractérisé en ce que l'on utilise des inhibiteurs de lipoxygénase qui sont choisis parmi les inhibiteurs redox et non redox, les précurseurs d'inhibiteurs redox, les agents anti-oxydants, les agents chélateurs du fer, les composés imidazolés, les phénothiazines, les dérivés du benzopyrane, les éicosanoïdes inhibiteurs, les produits interférant avec les flux calciques et en particulier les phénothiazines et les diphénylbutylamines, le vérapamil, le fuscoside, le curcumin, l'acide chlorogénique, l'acide caféique, l'acide 5,8,11,14-éicosatétrayénoïque (ETYA), l'hydroxyphénylrétinamide, le lonapalène, l'esculin, le diéthylcarbamazine, la phénantroline, la baicaléine, le proxicromil, les thioéthers et en particulier le sulfure de diallyle et le sulfure de di-(1 propényl).

5. Utilisation selon la revendication 4, caractérisé en ce que les inhibiteurs redox sont choisis parmi les dérivés des butanes catécholiques.

6. Utilisation selon la revendication 5, caractérisé en ce que les dérivés des butanes catécholiques sont choisis parmi l'acide nordihydroguaiarétique (NDGA), le masoprocol.

7. Utilisation selon la revendication 4, caractérisé en ce que les inhibiteurs redox sont choisis parmi la phénidone, le lanopalen, les indazolinones, le naphazatrom, le benzofuranol, l'alkylhydroxylamine, les composés de formules suivantes:

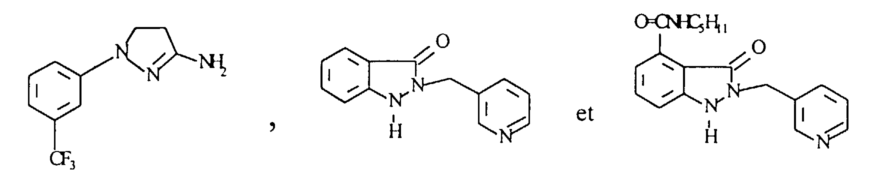

8. Utilisation selon la revendication 4, caractérisé en ce que les inhibiteurs non redox sont choisis parmi les hydroxythiazoles, les méthoxyalkylthiazoles, les benzopyranes et leurs dérivés, le méthoxytétrahydropyrane, les acides boswelliques et leurs dérivés acétylés, les acides quinolinméthoxyphénylacétiques substitués par des radicaux cycloalkyles.

9. Utilisation selon la revendication 4, caractérisé en ce que les agents antioxydants sont choisis parmi les phénols, le propylgallate, les fiavonoïdes et/ou les composés naturels contenant de tels flavonoïdes, en particulier les dérivés hydroxylés des flavones tels que le flavonol, la dihydroquercétine, la lutéoline, la galangine, l'orobol, les dérivés du chalcone tel que le 4,2',4'-trihydroxychalcone, les orthoaminophénols, les N-hydroxyurées, les benzofuranols, l'ebselen et les agents susceptibles d'accroître l'activité des séléno-enzymes réductrices.

10. Utilisation selon la revendication 4, caractérisé les agents chélateurs du fer sont choisis parmi les acides hydroxamiques et leurs dérivés, les N-hydroxyurées, le 2-benzyl-1-naphtol, les catéchols, les hydroxylamines, le carnosol trolox C, le catéchol, le naphtol, la sulfasalazine, le zyleuton, l'acide 5-hydroxyanthranilique et les acides 4-(oméga-aryllalkyl)phénylalcanoïques.

11. Utilisation selon la revendication 4, caractérisé en ce que les composés imidazolés sont choisis parmi le kétoconazole, l'itraconazole.

12. Utilisation selon la revendication 4, caractérisé en ce que les éicosanoïdes inhibiteurs sont choisis parmi les acides octa-décatétraénoïque, éicosatétraénoïque, docosapenténoïque, eicosahexaénoïque, docosahexaénoïque, et leurs différents esters, de même que divers autres éicosanoïdes, éventuellement sous forme d'esters, tels que PGE1 (prostaglandine E1), PGA2 (prostaglandine A2), le viprostol, les acides 15-monohyd roxyéicosatétraénoique, 15-monohydroxyéicosatriénoique et 15-monohydroxyéicosapentaénoique, les leukotriènes B5, C5 et D5.

13. Utilisation selon l'une quelconque des revendications précédentes, caractérisé en ce que l'on utilise des inhibiteurs de cyclo-oxygénase qui sont choisis parmi les agents anti-inflammatoires non stéroïdiens.

14. Utilisation selon la revendication 13, caractérisé en ce que les agents anti-inflammatoires non stéroïdiens sont choisis parmi les dérivés arylcarboxyliques, les dérivés pyrazolés, les dérivés de l'oxicam, les dérivés de l'acide nicotinique.

15. Utilisation selon l'une quelconque des revendications 13 ou 14, caractérisé en ce que l'inhibiteur de cyclo-oxygénase est choisi parmi l'acide méclofenamique (5659, Merck index, 11ième édition), l'acide méfénamique (5680, Merck index, 11ième édition), le carprofen (1870, Merck index, 11ième édition), le diclofenac (3071, Merck index, llième édition), le diflunisal (3130, Merck index, 11ième édition), le fenbufen (3906, Merck index, 11ième édition), le fenoprofen (3926, Merck index, 11ième édition), l'ibuprofen (4812, Merck index, 11ième édition), l'indométhacine (4874, Merck index, 11ième édition), le ketoprofen (5184, Merck index, 11ième édition), la nabumetone (6258, Merck index, 11ième édition), le naproxen (6337, Merck index, 11ième édition), le sulindac (8961, Merck index, 11ième édition), le tenoxicam (9080, Merck index, 11ième édition), la tolmetine (9441, Merck index, 11ième édition), ou encore l'acide acétylsalicylique.

16. Utilisation selon l'une quelconque des revendications précédentes, caractérisé en ce que l'inhibiteur de lipoxygénase est utilisé à une concentration allant de 0,01% à 5 % en poids et de préférence de 0,1 % à 1 %, plus particulièrement de 0,1 % à 0,8 %.

17. Utilisation selon l'une quelconque des revendications précédentes, caractérisé en ce que l'inhibiteur de cyclo-oxygénase est utilisé à une concentration allant de 0,001 % à 5 % en poids et de préférence de 0,01% à 0,1%.

18. Composition, caractérisée en ce qu'elle comprend au moins deux composés distincts, l'un au moins étant un inhibiteur de lipoxygénase et l'autre étant au moins un inhibiteur de cyclo-oxygénase.

19. Composition selon la revendication 18, caractérisée en ce qu'elles sont conditionnées sous une forme convenant à une application topique.

20. Composition selon l'une quelconque des revendications 18 ou 19, caractérisée en ce que les inhibiteurs des lipoxygénases et des cyclo-oxygénases sont tels que définis aux revendications 4 à 15.

21. Composition selon l'une quelconque des revendications 18 à 20, caractérisée en ce que les inhibiteurs des lipoxygénases et des cyclo-oxygénases sont à des concentrations telles que définis aux revendications 16 ou 17.

22. Composition selon l'une quelconque des revendications 18 à 21, utilisée à titre de médicament.

23. Composition selon la revendication précédente, caractérisée en ce que le médicament est destiné à favoriser la chute des poils et/ou des cheveux ou à ralentir et/ou empêcher la pousse des poils et/ou des cheveux.

24. Dispositif à plusieurs compartiments ou "kits", caractérisé en ce qu'il comprend dans un premier compartiment au moins un inhibiteur de lipoxygénase et dans un deuxième compartiment au moins un inhibiteur de cyclo-oxygénase.

25. "Kits" selon la revendication 24, caractérisé en ce que les composés rentrant dans lesdits premier et deuxième compartiments sont conditionnés sous une forme convenant à une application topique.

26. "Kits" selon l'une des revendications 24 ou 25, caractérisé en ce que les inhibiteurs des enzymes lipoxygénases et cyclo-oxygénases sont tels que définis aux revendications 4 à 17.

27. Procédé de traitement non thérapeutique pour modifier la pousse des poils et/ou des cheveux caractérisé en ce qu'il comprend une étape d'administration à l'organisme, par voie topique et/ou systémique, d'au moins un inhibiteur de lipoxygénase et d'au moins un inhibiteur de cyclo-oxygénases.

28. Procédé selon la revendication 27, caractérisé par le fait que les inhibiteurs sont administrés par voie topique.

29. Procédé selon l'une des revendications 27 ou 28, caractérisé en ce que l'on applique sur la peau et/ou le cuir chevelu des compositions à usage topique contenant conjointement les inhibiteurs de lipoxygénase et de cyclo-oxygénase.

30. Procédé selon l'une des revendications 27 à 29, caractérisé en ce que les inhibiteurs de lipoxygénase et cyclo-oxygénase sont tels que définis aux revendications 4 à 15.

31. Procédé selon l'une des revendications 27 à 30, caractérisé en ce que les inhibiteurs de lipoxygénase et cyclo-oxygénase sont à des concentrations telles que définis aux revendications 16 ou 17.
